# EUROPEAN PATENT SPECIFICATION

(11) **EP 0 215 274 B1**
(45) Date of publication and mention of the grant of the patent: **31.03.1993**
(21) Application number: 86110914.8
(22) Date of filing: 07.08.1986
(51) Int. Cl.: C07G 17/00, C12P 1/00, C12N 5/00, C12N 1/38, A61K 35/36, A61K 7/06

(54) **Epidermal cell extracts and method to enhance wound healing and regenerate epidermis**
Epidermiszellenextrakte und Verfahren zur Verbesserung der Wundheilung und zur Regenerierung der Epidermis
Extraits de cellules épidermiques et procédé pour améliorer la guérison des plaies et pour régénérer l'épiderme

(30) Priority: 14.08.1985 US 765711
(43) Date of publication of application: 25.03.1987
(73) Proprietor: Memorial Hospital for Cancer and Allied Diseases, New York, N.Y. 10021 (US)
(72) Inventor: Eisinger, Magdalena, Demarest New Jersey 07627 (US)
(74) Representative: VOSSIUS & PARTNER

(56) References cited:
- EP-A- 0 013 394
- WO-A-81/00260
- US-A- 4 254 226
- BIOLOGICAL ABSTRACTS, vol. 70, 1980, no. 22767, Philadelphia, PA, US; A.R. JOHNSON et al.: "Growth-promoting actions of extracts from mouse submaxillary glands on human endothelial cells in culture", && CELL PHYSIOL 101(3) 431;-438 1979
- CHEMICAL ABSTRACTS, vol. 79, no. 7, 20th August 1973, page 117, no. 39560p, Columbus, Ohio, US; S.W. MELBYE et al.: "Characteristics of a factor stimulating skin epithelial cell growth in vitro", & EXP. CELL RES. 1973, 79(2), 279-86
- Trends Biochem. SCI. 4 : 132-134 (1979)

## Description

This invention concerns enhancement of wound healing and coverage by epidermal cells using a physiologically active epidermal cell extract.

Epidermal cells grown in vitro are extracted by methods known in the art. Supernatants of these extracts can be applied to open wounds to speed healing. This material will also speed growth of epidermal cells or sheets of epidermal cells in culture.

Normal undamaged skin is composed of the epidermis and the dermis. Epidermis consists of an inner layer of viable nucleated cells covered by laminated cornified cells without nuclei. It contains nerve fibrils, but not blood vessels. The basal layer of the epidermis has irregular ridges (rete ridges) molded against the underlying connective tissue (dermal papillae). The dermis consists of collagen bundles and fibers containing many fixed tissue cells and is richly vascularized by capillaries and venules. Hair follicles, sweat glands and sebaceous glands extend from the dermis to the surface of the skin; such glands and follicles are lined by epithelial cells. Underneath the dermis there is an underlying layer of fatty tissue.

The terminology used in the description of burn wounds will be used. First degree of burn is superficial, it may remove most of the epidermis and part of the dermis. Because of the uneven interface between epidermis and dermis it may leave clusters of intact epidermal cells interdispersed in the damaged area.

Second degree of burn is deeper and may remove all epidermal cells, without destroying the epithelial cells, that line the hair follicles, sebaceous and sweat glands. If this occurs, epithelial cells from the hair follicles and glands can proliferate and migrate over the wound, providing a shallow layer of epidermis. Such a layer is often irregular and fragile and may impede the proper healing of the wound.

Third degree burn is the deepest and destroys all epidermis and dermis including their follicles and sebaceous and sweat glands. It is also termed "full thickness" wound.

Loss of epidermal coverage by injury or as a consequence of a disease process leaves an open wound which is vulnerable to infection. If this loss is extensive it also results in excessive loss of fluids and impairment of thermal regulation. Therefore, all clinical effort is concentrated on achieving the most speedy wound closure. Current methods of treatment are as follows: In burn patients, where a large body area is damaged the surgeons are using specially prepared pig skin (xenografts) to provide a mechanical barrier. These xenografts can be kept on the patients for 3-5 days. However, they are rejected it kept longer, leaving again an open wound. Homografts (allografts) can be obtained from human donors (cadavers). However, they are in a short supply and they are also rejected after a brief period of time.

The most effective current treatment is the use of autografts. These partial thickness sections of the skin are removed from an undamaged area of the patient and transplanted onto the wounded area. They become permanently attached and proliferate. If the wounded area is extensive and the skin available is limited, burn surgeons use small patches of skin (approximately 5 x 3 cm) spaced at a distance of 3-5 cm apart. These areas are eventually covered by the outgrowth of epidermis from the transplanted pieces of split thickness skin.

The removal of the skin for grafting is usually performed with an instrument called a dermatone. The thickness of the removed skin can be controlled. Because of the papillary nature of the epidermis, even with the thinnest cut all epidermis and some of the dermis is removed by this procedure. This harvesting operation is a painful, invasive process and causes scarring. Autografts may be repeatedly harvested form the donor site, after its healing.

Autografts, in order to cover a larger area can be also meshed by a device which slits the skin in a regular pattern and enlarges the piece of skin approximately 10 fold. The space caused by slits is eventually filled by cells growing from the sides.

All these modes of treatment are time consuming, costly and lead to various problems such as hypertrophic scarring and contracture.

The newest experimental approach to wound treatment is the use of autologous or allogeneic cells grown in tissue culture. In this method a small piece of skin is removed from the patients and grown in tissue culture. One of such methods to grow epidermal cells in tissue was described by us (Eisinger et al., U.S. patent No. 4,254,226 issued March 3, 1981) and used for wound treatment (see U.S. Patent No. 4,229,819 issued November 10, 1981). The enlarged piece of epidermis is than applied to the same patient as a new sheet of cells.

We have recently found that an epidermal cell extract prepared from human epidermal cells grown in vitro can be successfully used for enhancement of wound coverage by epidermal cells and regeneration of epidermis.
Thus, the present invention relates to an aqueous, cell-free extract from cultured human epidermal cells capable of stimulating growth and regeneration of epidermal cells. It further relates to the use of an aqueous, cell-free extract derived from cultured human epidermal cells for the manufacture of a composition useful for stimulating the growth or regeneration of epidermal cells in a subject whose epidermal cells are not growing or regenerating adequately.

Preparation of epidermal cell extracts involves: Growth of epidermal cells in vitro; and extraction of the factor(s) that promote epidermal cell growth.

### Example I

Human Epidermal cells were grown in vitro by methods originally described by us (Eisinger M, et al., Human epidermal cell cultures: Growth and differentiation in the absence of dermal components or medium supplements, Proc. Nat'. Acad. Sci. USA 76:5340-5344, 1979) and also as described in a United States Patent (No. 4,254,226 issued March 3, 1981). The procedure is as originally described in the publication and the above patent except that it has now been found that the preferred pH range is 5.9-6.6.

### Example II

Preparation of the epidermal cell extract:
Confluent cultures of epidermal cells were washed twice with phosphate buffered saline and scraped using a rubber policeman. The cells were pelleted and resuspended in 1:1 v/v in a phosphate buffered saline (PBS) and sonicated twice for 15 seconds, diluted 1:10 with phosphate buffered saline and clarified by two-step centrifugation at 16,000 x g for 20 minutes and 150 x g for 45 minutes. Supernatants so derived contained growth stimulating and wound healing factor(s). They can be kept frozen at -20°C or -80°C.

### Example III

Application of the epidermal cell extracts:
Clarified supernatants which contain epidermal cell growth promoting and differentiation factor(s) can be applied to an open wound using an adsorbing material (we used Release, a non-adhesive dressing manufactured by Johnson & Johnson). This procedure could well be applied for the healing of second degree burns or any wounds which are equivalent in their depth to a deep second degree burn. In addition, the material can be lyophilized and used either in the form of a spray, powder or mixed into a cream for skin treatment. It is also possible to use the material within a matrix to cover the wound in vivo; such as collagen matrix or a sponge to keep the wound moist. One can also spray the material onto the wound in vivo or onto the cells in vitro.

Four experimental animals (pigs) were wounded by removing the skin with a dermatome at 0.1 cm (40/1000 of an inch). This corresponds to a deep second degree burn and removes all the epidermis and most of the dermis. Deep hair follicles and associated glandular structures were left in the wound bed. The wounds were approximately 15.24 x 7.62 cm (6 inches by 3 inches). There were 2-3 dorsal lateral wounds per animal. Such wounds in pigs when covered with a dressing and kept bandaged will be resurfaced with functional epithelium in approximately 10-14 days. Since many hair follicles are damaged by wounding, the reepithalized wound has very little hair. We have found, that by using the described growth factor, the wounds were functionally covered in 6-7 days, and fully covered by hair. By histological examinations read by a pathologist at 5-7 days post wounding it was found, that the factor stimulated epithelial cells of remaining hair follicles and other presumably precursor cells in the dermis to migration and replication. Three times as many cells are found to be in mitosis in the area treated by the factor, than in the control wound. This resulted in an epidermal coverage of the wound at first thicker than normal pig epidermis. This hyperplasia ceased in approximately 3 weeks after the application of the factor, and epidermis had a normal appearance. The effect of the factor depends on the concentration used. The dilution used for treatment is 1/100 dilution in PBS. When the dilution is 1/200, healing time is approximately 8-10 days. There was no immunological response observed, i.e. no lymphocyte infiltration, or inflammation or other sign of immunological response was found at the site of the wound to which the factor was applied over a period of 4-6 weeks.

### Example IV

Experiments in tissue culture, using pig or human epidermal cells in culture, confirmed the observations of Example III above in vivo. It was found, that either of these types of epidermal cells grown or plated in the presence of the factor extracted from either human or ovine (pig) epidermal cells had a better plating efficiency and they formed colonies of rapidly growing cells even when plated at low seeding densities (1 x 10⁴ cells/60 mm Petri dish) at this cell density. Controls in the absence of the factor did not grow. When plated at optimal seeding densities (5 x 10⁵/60 mm Petri dish) they also started replicating much earlier than in its absence or in the presence of an extract from other cells such as WI-38.

### Example V

On a full thickness wound bed, corresponding a IIIrd degree burn, there are no epithelial remnants, and therefore epidermal cells have to be introduced into the wound. For this purpose small pieces of autologous skin, or skin grown in tissue culture can be used. Simultaneous application of the factor will enhance the outgrowth of epidermal cells from transplanted epidermal cells and enhance full coverage of the wounded area.

The above examples are for illustrative purposes only and are not meant to limit the invention to the specific examples shown. It will be obvious to those skilled in the art that these epidermal cell extracts can be obtained in general from mammalian epidermal cells for use on other mammlian epidermal or epithelial cells in vivo or in vitro. It will also be obvious to those skilled in the art that any medium that supports the growth of epidermal cells in culture can be used; also any method known in the art can be used to extract the epidermal cell growth factor(s) such as freeze-thawing, sonication etc. The cells used for the extract can be autologous, allogeneic or xenogeneic to the treated recipient as shown in the examples. Also methods known in the art can be used to bring down particulate matter from the broken cells and leave the active epidermal growth enhancement factor in the supernatant. Any diluent which will not harm the potency of the extract can be used as well.

This material can be used for wounds or burns to enhance healing and reduce the covering time of same and reduce resulting infection and trauma. It may also reduce the healing time of surgical incision. These epidermal extracts will speed the growth of epithelial cells in culture to provide burn victims with autologous tissue grafts factor thus reducing the post-burn syndromes of infection and shock. One can also speed growth of an allogenic transplant or a xenogeneic cell transplant.

The invention we are describing will substantially speed the healing of the first and second degree wound, will also speed outgrowth of the pieces of autologous split thickness skin and of the skin prepared in tissue culture which are applied to a third degree wound.

The material has cosmetic use as well to remove or ameliorate the age spots on skin. The extract material can be used to provide skin regeneration in situ. Its effects may also lead to hair growth. Internally this material may be useful to heal chronic wounds such as ulcers, or blistering skin disease as epidermolysis bullosa.

## Claims

1. Aqueous, cell-free extract from cultured human epidermal cells capable of stimulating growth and regeneration of epidermal cells.

2. The use of an aqueous, cell-free extract derived from cultured human epidermal cells for the manufacture of a composition useful for stimulating the growth or regeneration of epidermal cells in a subject whose epidermal cells are not growing or regenerating adequately.

3. The use of claim 2, wherein the subject's epidermal cells are contacted with an adsorbing material and/or a matrix carrying the cell-free extract derived from epidermal cells.

4. The use of claim 2, wherein the subject is a mammal.

5. The use of claim 4, wherein the mammal is a human or swine.

6. The use of an aqueous, cell-free extract from cultured human epidermal cells for the manufacture of a composition useful for enhancing wound coverage and healing in a wounded subject.

7. The use of an aqueous, cell-free extract from cultured human epidermal cells for the manufacture of a composition useful for enhancing the wound coverage of transplanted skin material situated on a wounded region of a subject.

8. The use of claim 7, wherein the transplanted skin material is autologous relative to the subject.

9. The use of claim 7, wherein the transplanted skin material is allogeneic relative to the subject.

10. The use of claim 7, wherein the transplanted skin material is tissue culture grown epidermal cells.

11. A composition capable of stimulating epidermal cell growth, derived from an aqueous, cell-free extract of claim 1.

12. A pharmaceutical composition comprising an amount of the aqueous, cell-free extract of claim 1 or or 11, effective to stimulate growth and regeneration of epidermal cells and a pharmaceutically acceptable carrier.

13. A wound dressing comprising an effective wound healing amount of the composition of claim 1 or 11 and an adsorbing material and/or a matrix carrying the composition.

## Patentansprüche

1. Wäßriger, zellfreier Extrakt von gezüchteten menschlichen Epidermiszellen, der fähig ist zur Stimulation des Wachstums und der Regeneration von Epidermiszellen.

2. Verwendung eines wäßrigen, zellfreien Extrakts, der von gezüchteten menschlichen Epidermiszellen stammt, für die Herstellung eines Mittels, das für die Stimulation des Wachstums oder der Regeneration von Epidermiszellen in einem Individuum geeignet ist, dessen Epidermiszellen nicht wachsen oder sich nicht hinreichend regenerieren.

3. Verwendung nach Anspruch 2, wobei die Epidermiszellen des Individuums mit einem adsorbierenden Material und/oder einer Matrix in Kontakt gebracht werden, die den von Epidermiszellen stammenden zellfreien Extrakt tragen.

4. Verwendung nach Anspruch 2, wobei das Individuum ein Säuger ist.

5. Verwendung nach Anspruch 4, wobei der Säuger ein Mensch oder Schwein ist.

6. Verwendung eines wäßrigen, zellfreien Extrakts von gezüchteten menschlichen Epidermiszellen für die Herstellung eines Mittels, das für die verbesserte Wundbedeckung und -heilung in einem verwundeten Individuum geeignet ist.

7. Verwendung eines wäßrigen, zellfreien Extrakts von gezüchteten menschlichen Epidermiszellen für die Herstellung eines Mittels, das für die Verbesserung der Wundbedeckung von transplantiertem Hautmaterial geeignet ist, das sich auf einem Wundbereich eines Individuums befindet.

8. Verwendung nach Anspruch 7, wobei das transplantierte Hautmaterial autolog in Bezug auf das Individuum ist.

9. Verwendung nach Anspruch 7, wobei das transplantierte Hautmaterial allogen in Bezug auf das Individuum ist.

10. Verwendung nach Anspruch 7, wobei das transplantierte Hautmaterial in einer Gewebekultur gewachsene Epidermiszellen sind.

11. Mittel, das zur Stimulation des Wachstums von Epidermiszellen fähig ist, welches von einem wäßrigen, zellfreien Extrakt gemäß Anspruch 1 stammt.

12. Arzneimittel, enthaltend eine Menge des wäßrigen zellfreien Extrakts nach Anspruch 1 oder 11, die eine Stimulation des Wachstums und der Regeneration von Epidermiszellen bewirkt, und einen pharmazeutisch verträglichen Träger.

13. Wundverbandmaterial, umfassend eine wirksame wundheilende Menge des Mittels nach Anspruch 1 oder 11 und ein adsorbierendes Material und/oder eine Matrix, die das Mittel tragen.

## Revendications

1. Extrait aqueux, exempt de cellules, provenant de cellules épidermiques humaines cultivées, capable de stimuler la croissance et la régénération des cellules épidermiques.

2. Utilisation d'un extrait aqueux, exempt de cellules, dérivé de cellules épidermiques humaines cultivées, pour la préparation d'une composition utilisable pour stimuler la croissance ou la régénération des cellules épidermiques chez un sujet dont les cellules épidermiques ne croissent pas ou ne se régénèrent pas de manière adéquate.

3. Utilisation selon la revendication 2, dans laquelle les cellules épidermiques du sujet sont mises en contact avec un matériau adsorbant et/ou une matrice portant l'extrait exempt de cellules dérivé des cellules épidermiques.

4. Utilisation selon la revendication 2, dans laquelle le sujet est un mammifère.

5. Utilisation selon la revendication 4, dans laquelle le mammifère est un humain ou un porc.

6. Utilisation d'un extrait aqueux, exempt de cellules, provenant de cellules épidermiques humaines cultivées, pour la préparation d'une composition utilisable pour augmenter le recouvrement des plaies et la cicatrisation chez un sujet blessé.

7. Utilisation d'un extrait aqueux, exempt de cellules, provenant de cellules épidermiques cultivées, pour la préparation d'une composition utilisable pour augmenter le recouvrement des plaies d'un matériau cutané transplanté situé sur une région blessée d'un sujet.

8. Utilisation selon la revendication 7, dans laquelle le matériau cutané transplanté est autologue par rapport au sujet.

9. Utilisation selon la revendication 7, dans laquelle le matériau cutané transplanté est allogène par rapport au sujet.

10. Utilisation selon la revendication 7, dans laquelle le matériau cutané transplanté est constitué par des cellules épidermiques développées en culture de tissu.

11. Composition capable de stimuler la croissance des cellules épidermiques, dérivée d'un extrait aqueux, exempt de cellules, selon la revendication 1.

12. Composition pharmaceutique comprenant une quantité d'extrait aqueux, exempt de cellules, selon la revendication 1 ou 11, efficace pour stimuler la croissance et la régénération des cellules épidermiques et un véhicule acceptable du point de vue pharmaceutique.

13. Pansement pour plaie comprenant une quantité efficace pour la cicatrisation de la composition selon la revendication 1 ou 11 et un matériau adsorbant et/ou une matrice portant la composition.
